# EUROPEAN PATENT SPECIFICATION

(11) **EP 3 645 139 B1**
(45) Date of publication and mention of the grant of the patent: **26.07.2023**
(21) Application number: 18711827.8
(22) Date of filing: 23.02.2018
(51) Int. Cl.: B01D 15/18, B01D 15/34, C07K 1/16

(54) **PERIODIC COUNTERCURRENT CHROMATOGRAPHY SEPARATION OF PLASMIDS**
PERIODISCHE GEGENSTROMCHROMATOGRAPHIETRENNUNG VON PLASMIDEN
SÉPARATION PAR CHROMATOGRAPHIE À CONTRE-COURANT PÉRIODIQUE DE PLASMIDES

(30) Priority: 26.06.2017 GB 201710130
(43) Date of publication of application: 06.05.2020
(73) Proprietor: Cytiva BioProcess R&D AB, 751 84 Uppsala (SE)
(72) Inventor: BLOM, Hans, 751 84 Uppsala (SE); AKERBLOM, Anna, 751 84 Uppsala (SE); SKOGLAR, Helena, 751 84 Uppsala (SE); SENDABO, Sara, 751 84 Uppsala (SE)
(74) Representative: Démoulin, Eva Lotta
(86) International application number: PCT/EP2018/054478
(87) International publication number: WO 2019/001778

(56) References cited:
- EP-A1- 3 118 620
- WO-A1-2008/153472
- FR-A1- 2 929 533
- US-A1- 2013 248 451
- US-A1- 2013 280 788

## Description

### Technical field of the invention

The present invention relates to separation processes in the manufacturing of biopharmaceuticals, and more particularly to a separation process useful for separation of large species such as plasmids, virus particles etc.

### Background of the invention

In the manufacturing of biopharmaceuticals such as vaccines, antibodies, recombinant proteins, gene therapy vectors etc. several chromatographic separation steps are usually needed to remove various contaminants and impurities from the product. These separation steps add significant cost and process time and there is hence a significant interest in intensifying them.

Multicolumn continuous chromatography processes are available, where the feed is applied to a first column and is then diverted to one or more subsequent columns as the first columns approaches saturation and the first column is eluted and regenerated to be loaded again during elution and regeneration of the subsequent column(s). Such processes can be denoted periodic countercurrent chromatography (PCC) or simulated moving bed (SMB) and are of considerable interest for separation of biopharmaceuticals, see e.g. US7901581, US20130248451, US20130280788 and US7220356.

PCC/SMB processes can significantly increase the productivity, but the increases achieved can depend quite strongly on the design of the process.

Plasmids are of interest in the biopharmaceutical area primarily as vectors for gene therapies and certain cell therapies (e.g. for preparing CAR-T cells) but also in vaccine technology. The plasmids are large ring-shaped DNA molecules of bacterial origin, which present particular issues in chromatographic processes as their size prevents them from entering the pores of most chromatography media. A common first step in plasmid purification is a so-called group separation, where the plasmid preparation is applied to a gel filtration resin and the plasmid is recovered in the void fraction or a slightly retained fraction, while RNA, a major contaminant, enters the pores of the resin and is significantly retained. As with other gel filtration processes, this is a slow process with limited loading capacity and it would be highly interesting to speed it up by applying continuous processing.

Accordingly, there is a need for new group separation processes for plasmids and other large species, such as e.g. virus particles, allowing an increased efficiency of the processes.

### Summary of the invention

One aspect of the invention is to provide an efficient method for continuously separating a plasmid or e.g. a virus particle or other large species. This is achieved with a method of applying a process feed containing a plasmid/virus particle or other large species to an apparatus with at least three chromatography columns packed with separation matrix particles, wherein while one chromatography column is loaded with the process feed, another chromatography column is eluted with an eluent to recover the separated plasmid, and yet another chromatography column is eluted with a further eluent to remove contaminants.

Further suitable embodiments of the invention are described in the dependent claims.

### Drawings

Fig. 1 shows an example of a chromatography system that can be used with the method of the invention.
Fig. 2 shows another example of a chromatography system that can be used with the method of the invention.
Fig. 3 shows an example of the method of the invention using three columns.
Fig. 4 shows an example of the method of the invention using four columns.
Fig. 5 shows results from the single-column run 1 of Example 1.
Fig. 6 shows results from the single-column run 2 of Example 1.
Fig. 7 shows results from the four-column PCC run 1 of Example 2.
Fig. 8 shows results from the four-column PCC run 2 of Example 2.
Fig. 9 shows results (enlargement) from the four-column PCC run 2 of Example 2.
Fig. 10 shows an electrophoresis gel with 1 Mw markers, 2 the sample and 3 the plasmid fraction.

### Detailed description of embodiments

In one aspect, the present invention discloses a method of continuous separation of a plasmid from a process feed comprising a plasmid in an apparatus with at least three chromatography columns. The method can also be used for other separations of large species, e.g. viruses, virus-like particles, antigen conjugates etc., from lower molecular-weight contaminants. The columns are packed with separation matrix particles, and while one chromatography column is loaded with the process feed, another chromatography column is eluted with an eluent to recover the separated plasmid, and yet another chromatography column is eluted with a further eluent (which can be the same eluent as used for eluting the plasmid) to remove contaminants such as RNA. It can be advantageous if the flow rates in the different steps are matched, so that no standstill is required in any step to wait for another step to finish.

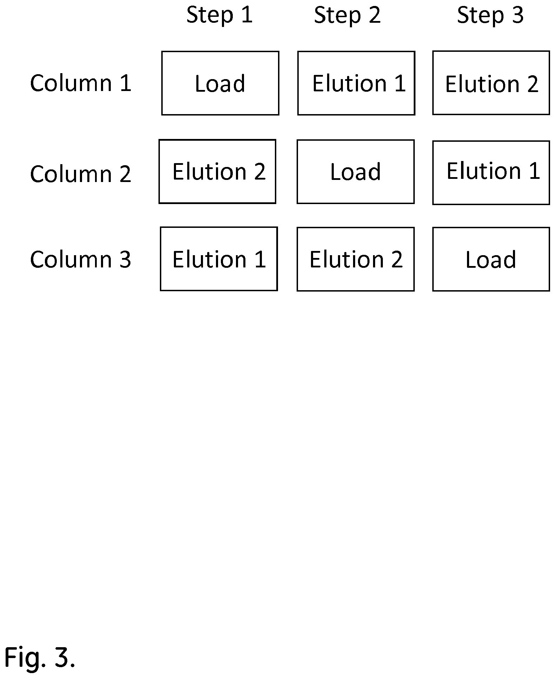

The method may in some embodiments, as illustrated by Fig. 3, comprise the steps of:
a) conveying a process feed comprising a plasmid through a first chromatography column comprising a packed bed of separation matrix particles;
b) conveying an eluent through the first chromatography column, recovering the eluent with the plasmid after passage of the first chromatography column; and conveying the process feed through a second chromatography column packed with the same separation matrix as the first chromatography column; and
c) conveying a further eluent (which can be the same eluent as in step b) through the first chromatography column; conveying an eluent through the second chromatography column, recovering the eluent with the plasmid after passage of the second chromatography column; and conveying the process feed through a third chromatography column packed with the same separation matrix as the first and second chromatography columns.

After step c), steps a)-c) may repeated for the duration of the continuous process, e.g. until the entire process feed has been processed. In these repetitions step a) may further comprise conveying a further eluent through the second chromatography column while conveying an eluent through the third chromatography column and recovering the eluent with the plasmid after passage of the third chromatography column; and step b) may further comprise conveying a further eluent through the third chromatography column. Suitably, a process feed or eluent is conveyed through all three columns throughout all of steps a)-c), i.e. no flows are stopped to wait for another column/step to finish.

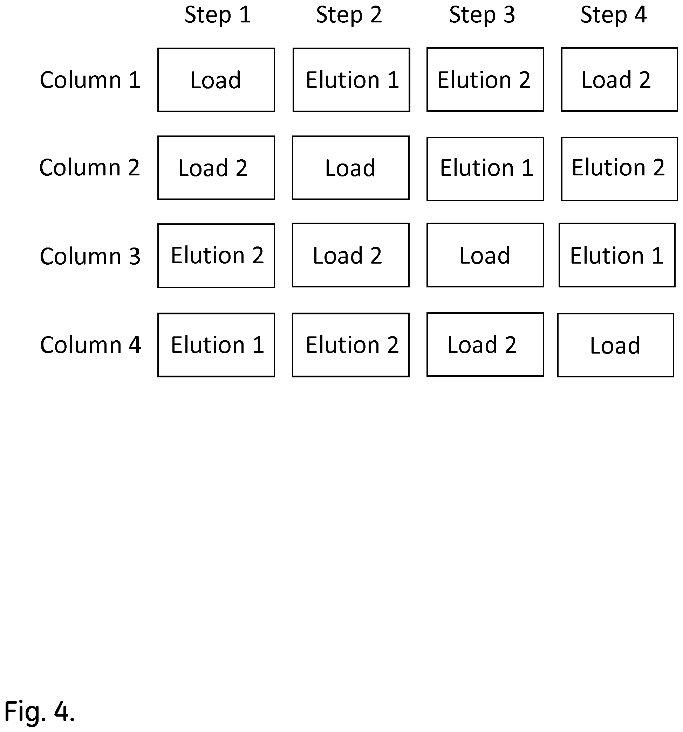

In certain embodiments, as illustrated by Fig. 4, the method comprises the steps of:
a) conveying a process feed comprising a plasmid through a first chromatography column comprising a packed bed of separation matrix particles, and conveying an outflow from the first chromatography column through a second chromatography column packed with the same separation matrix as the first column;
b) conveying an eluent through the first chromatography column, recovering the eluent with the plasmid after passage of the first chromatography column; and conveying the process feed through the second chromatography column and further through a third chromatography column packed with the same separation matrix as the first and second columns;
c) conveying a further eluent through the first chromatography column; conveying an eluent through the second chromatography column, recovering the eluent with the plasmid after passage of the second chromatography column; and conveying the process feed through the third chromatography column and further through a fourth chromatography column packed with the same separation matrix as the first, second and third columns; and
d) conveying a process feed comprising a plasmid through the fourth chromatography column and conveying an outflow from the fourth chromatography column through the first chromatography column; conveying a further eluent through the second chromatography column; and conveying an eluent through the third chromatography column, recovering the eluent with the plasmid after passage of the third chromatography column.

After step d), steps a)-d) may be repeated for the duration of the continuous process, e.g. until the entire process feed has been processed. In these repetitions, step a) may further comprise conveying a further eluent through the third chromatography column and conveying an eluent through the fourth chromatography column while recovering the eluent with the plasmid after passage of the fourth chromatography column; and step b) may further comprises conveying a further eluent through the fourth chromatography column. Suitably, a process feed or eluent is conveyed through all three columns throughout all of steps a)-d), i.e. no flows are stopped to wait for another column/step to finish.

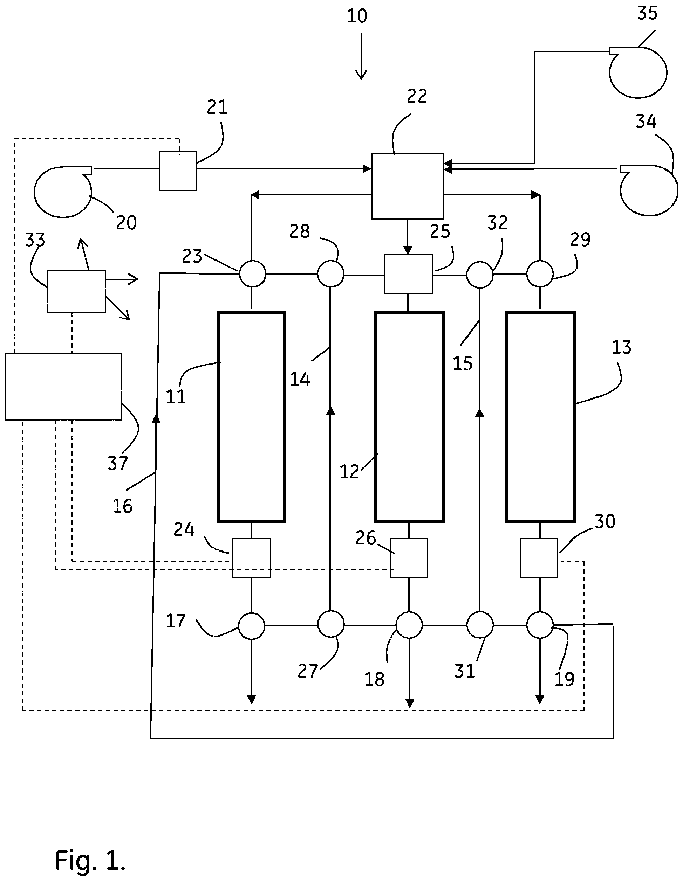

The method can e.g. be carried out in a chromatography system 10 as illustrated in Fig. 1. Such a system comprises a plurality of chromatography columns 11,12,13 (three columns are shown in Fig. 1 but four or more columns are equally possible). The system can suitably be arranged for performing continuous chromatography. The columns may be packed with the same separation matrix and connected with one or more connecting lines 14,15,16 such that liquid can flow from one column **11**,**12** to a subsequent one **12**,**13** and from a last column **13** to a first column **11** and each connecting line between two columns may comprise at least one on/off valve **17**,**18**,**19**, which may be three-way or four-way valves. These valves in conjunction with valves/valve blocks **23**,**25**,**29** also allow parallel operations of the columns, e.g. elution of one or more columns simultaneously with the loading of one or more other columns. The system may further comprise a feed pump **20**, e.g. connected via a first detector **21** to a first valve block **22** and one or more buffer pumps **34**,**35**, e.g. connected to valve block **22**. The first valve block **22** can further be connected to the inlet of a first column **11** via a first valve **23**. An outlet end of the first column **11** may be connected to a second valve **17** through a second detector **24**. The first valve block **22** can further be connected to the inlet of a second column **12** via a second valve or valve block **25**. An outlet end of the second column **12** can be connected to valve **18** via a third detector **26**. Furthermore, a valve **27** can be connected between valve **17** and valve **18**. Valve **27** can also be connected to a valve **28** which is also connected to valve **23** and the second valve block **25**. Hereby the effluent from the first column **11** can be directed to the inlet of the second column **12** through connecting line **14**, valves **17**, **27**, **28** and **25**. Furthermore the first valve block **22** can be connected to the inlet of a third column **13** via valve **29**. An outlet end of the third column **13** may be connected to valve **19** via a fourth detector **30**. Furthermore valve **31** can be connected between valve **18** and valve **19**. Valve **31** can also be connected to valve **32** which may also be connected to the second valve block **25** and valve **29**. Hereby the effluent from the second column **12** can be directed to the inlet of the third column **13** through connecting line **15**. The effluent from the third column **13** can be directed to the inlet of the first column **11** through connecting line **16** via valves **19** and **23** (alternatively it can be directed to a subsequent fourth column). Furthermore, the first, second, third and fourth detectors **21**, **24**, **26**, **30** may all be connected to a determining unit **37**. The determining unit can be adapted to use the detected signals from the detectors to determine breakthrough and saturation points for the three different columns. The determining unit **37** and all the valve blocks, valves and pumps may further be connected to a control unit **33** (all the connections are not shown in Fig. 1) which is adapted to control the chromatography system in terms of when to remove or add columns from/into the loading zone, change flow rates, start new elution steps, etc. The detectors **21**, **24**, **26**, **30** can e.g. be UV detectors. The control unit **33** may be configured to control the system according to breakthrough data obtained from the determining unit **37**. Alternatively, control unit **33** can use fixed predetermined step times for the switching operations.

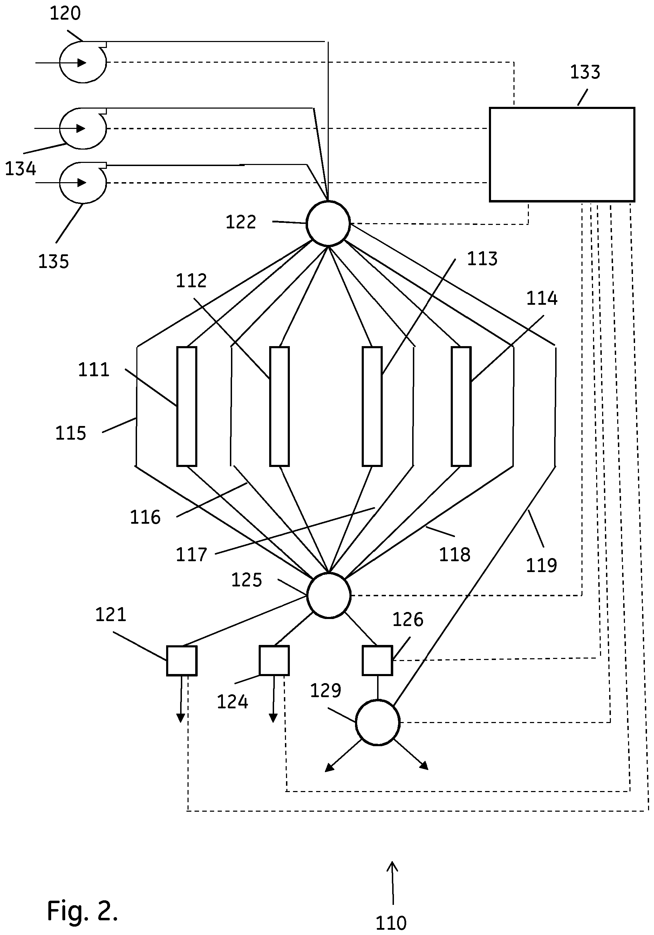

Fig. 2 illustrates an alternative chromatography system **110** for carrying out the method of the invention. The system comprises a plurality of chromatography columns (e.g. three or four columns) **111**,**112**,**113**,**114**, which may be packed with the same separation matrix. The columns are independently connectable to a feed pump **120**, buffer pumps **134**, **135** and/or connecting lines **115**, **116**, **117**, **118**, **119** via a multifunctional first valve block **122**. They are also independently connectable to the connecting lines and to the detectors **121**, **124**, **126** via multifunctional second valve block **125**. Valve blocks **122** and **125** allow the parallel coupling of several different flowpaths within the blocks (see e.g. WO2015094095 or WO2015094096 for useful designs of valves/valve blocks). They can also be used to connect one or more of the pumps with one or more of the connecting lines for bypassing or backflushing one or more of the columns. A further valve or valve block **129** allows the direction of a flow from detector **126** to either one or more collection vessels, waste or to connection line **119** for e.g. application on another column. Valves/valve blocks **122**,**125**,**129**, detectors **121**,**124**,**126** and pumps **120**,**134**,**135** are electrically or electromagnetically connected to a combined determining unit and control unit **133** for detection and for control of the flows. System **110** is similar to the commercially available ÄKTA^{™} pcc 75 system (GE Healthcare Life Sciences), with some minor modifications, e.g. the three pumps being directly connectable to three different columns. Both systems **10** and **110** may of course be further modified, e.g. by adding further columns, pumps, valves and/or detectors.

In the embodiments discussed above, the separation matrix particles can suitably be gel filtration particles having an exclusion limit for dextrans of less than 5000 kDa, such as of 500 kDa-5000 kDa or 500-3000 kDa. These particles enable the separation of a plasmid, which can often be in the size range of 2-20 kb (2-7 kb for vaccine-type plasmids and up to 20 kb for plasmids to be used in gene therapy), from lower molecular weight contaminants such as the more or less degraded RNA present in cell lysates, typically having a size of < 1 kb. The separation matrix particles may comprise a crosslinked polysaccharide, such as crosslinked agarose or alternatively crosslinked agar, cellulose or dextran. An example of crosslinked agarose particles can e.g. be Sepharose^{™} 6 Fast Flow beads (GE Healthcare Life Sciences) with a dextran exclusion limit of 2000 kDa, average particle diameter 90 µm and particle diameter range 45-165 µm. Alternatively, Sepharose CL-6B crosslinked agarose beads (GE Healthcare Life Sciences) with dextran exclusion limit approx. 1000 kDa may be used, although Sepharose 6 Fast Flow will allow higher flow rates due to its higher rigidity. To allow even higher flow rates, highly rigid crosslinked agarose beads may be used, e.g. beads as described in US6602990.

In some embodiments, the eluent (and optionally a further eluent) comprises at least 1.5 M of a salt, such as 1.5-2.5 M or 1.8-2.3 M salt. Alternatively expressed, the total salt concentration of the eluent (and optionally the further eluent) may be at least 1.5 M, such as 1.5-2.5 M or 1.8-2.3 M. The salt may e.g. be ammonium sulfate and the eluent/further eluent may e.g. comprise at least 1.5 M ammonium sulfate, such as 1.5-2.5 or 1.8-2.3 M ammonium sulfate. The presence of the salt/ammonium sulfate improves the separation between the plasmid and the RNA contaminants.

The process feed may comprise a clarified cell lysate, such as a lysate of gram negative cells, e.g. *E. coli.* In some embodiments, the method comprises, before the separation, a step 0) of preparing a clarified cell lysate as the process feed. Step 0) may comprise contacting cells, such as *E. coli* cells, with alkali and a surfactant, such as SDS, to induce lysis and neutralizing e.g. with potassium acetate. This is a well-known method, involving the addition of NaOH and SDS to final concentrations of e.g. 0.1 M and 0.05% (see e.g. L A Ciccolini et al: Biotech Bioeng 87, 293-302 (2004)). Step 0) may also be carried out in a continuous mode, e.g. by adding the lysis reagents in a static mixer or similar (see e.g. US2007/0213289).

The clarification can be done by traditional filtration, but to avoid excessive clogging of filters, it can be advantageous to remove the bulk of the particulates by flocculation/flotation before a final filtration. This can be achieved by adding a hydrogen carbonate, e.g. ammonium hydrogen carbonate, to the crude neutralised lysate at a pH around 5, such as at pH 4-6 or 4.5-5.5. If the pH is outside this interval, it may be adjusted after the hydrogen carbonate addition so as to fall within the interval. The carbon dioxide bubbles generated under these conditions will cause flotation of flocculated particulates to the surface, leaving an almost totally clarified lysate as the bulk liquid. Such a flotation process can also be performed continuously, e.g. by flowing the lysate through a flotation vessel where the flotated material on the surface is scraped off or otherwise diverted, while the clarified lysate is passed through a filter without any clogging issues.

In addition to the steps discussed above, the method may further comprise, after the separation, a step e) of purifying the recovered plasmid. This can be done e.g. by bind-elute chromatography and may suitably involve separating the desired super-coiled (sc) plasmid from open circle (oc) and other conformations of the plasmid. Both thiophilic and hydrophobic interaction chromatography methods can be used for this purpose. For thiophilic chromatography, resins like PlasmidSelect^{™} or PlasmidSelect Xtra (GE Healthcare Life Sciences) may be used, while various chromatographic resins with e.g. phenyl, butyl or hexyl groups have been suggested for hydrophobic interaction chromatography of plasmids (see e.g. US2007/0213289). Step e) can also be performed in a continuous mode, e.g. by using PCC or SMB continuous chromatography techniques. This can be achieved by the direct application of eluate from the steps above to a second multicolumn chromatography system. Alternatively, in a system with several chromatography columns, some columns may be used for the initial separation and some columns may be used for the further purification. In this case, the system may comprise both columns packed with gel filtration resin and columns packed with e.g. thiophilic or hydrophobic interaction resin.

### EXAMPLES

### Example 1 - batch chromatography on a single column

Sample: *E. coli* clarified lysate, containing plasmid pJV4 (6 kb), 17 mM Tris, 3.3 mM EDTA, 1 M potassium acetate.
Column: 20 ml Sepharose 6 Fast Flow (Sepharose 6FF) gel filtration resin (GE Healthcare Life Sciences) packed in a HiPrep^{™} 16/10 column (GE Healthcare Life Sciences), with 16 mm bed diameter and 100 mm bed height.
Chromatography system: ÄKTA pcc 75 system (GE Healthcare Life Sciences)
Equilibration buffer: 2.1 M ammonium sulfate, 10 mM EDTA, 100 mM Tris-HCl pH 7.5. Elution buffer: 2.1 M ammonium sulfate, 10 mM EDTA, 100 mM Tris-HCl pH 7.5.

### Run 1

**Table 1. Conditions for run 1.**

| Step | Buffer | Column volumes | Flow rate (ml/min) | Flow velocity (cm/h) | Residence time (min) |
|---|---|---|---|---|---|
| Equilibration | Equilibration buffer | 5 | 1.67 | 50 | 12 |
| Load | Sample | 0.3 | 1.67 | 50 | 12 |
| Elution | Elution buffer 1 | 3 | 1.67 | 50 | 12 |

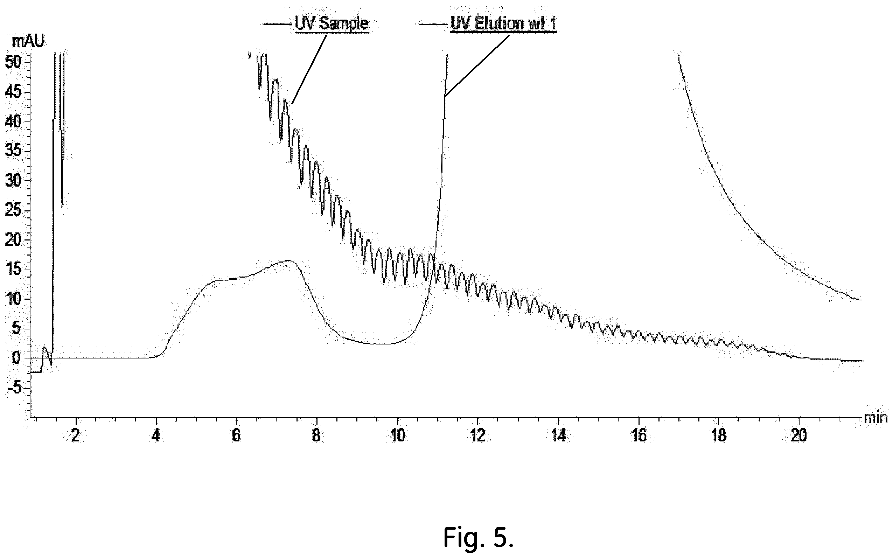

This was the initial run, for checking the separation pattern and starting optimisation. The results are shown in Fig. 5 and indicate a clear separation between the plasmid (the first peak) and contaminant RNA (the second peak).

### Run 2

**Table 2. Conditions for run 2.**

| Step | Buffer | Column volumes | Flow rate (ml/min) | Flow velocity (cm/h) | Residence time (min) |
|---|---|---|---|---|---|
| Equilibration | Equilibration buffer | 2 | 3.0 | 90 | 6.7 |
| Load | Sample | 0.3 | 3.0 | 90 | 6.7 |
| Elution | Elution buffer 1 | 3 | 3.0 | 90 | 6.7 |

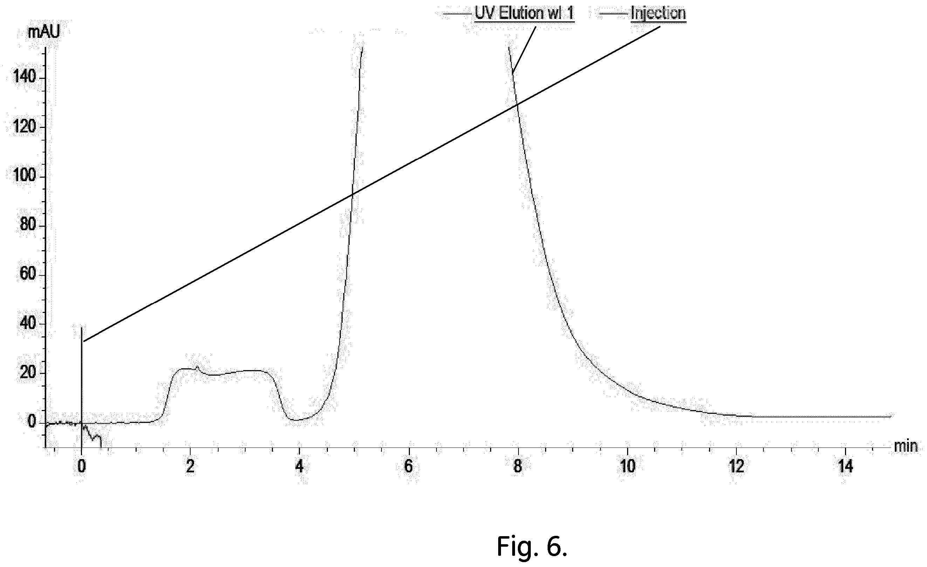

The flow rates were increased in comparison with run 1. As shown in Fig. 6, the shape of the plasmid peak is still similar to run 1. The plasmid peak has a volume of 7.7 ml, corresponding to 0.39 column volumes, which indicates a certain degree of dilution.

### Example 2 - 4-column PCC experiments

A periodic countercurrent chromatography (PCC) setup with four columns of the same type as used in Example 1 was arranged in the ÄKTA pcc 75 chromatography system. The sample and the buffers were the same as in Example 1, although the equilibration step was only performed once for all the columns before starting the PCC cycles. Fig. 4 shows an overview of the different columns during the process. For the loading step, two serially coupled columns were used so that the outflow from the first loading column (Load 1) was directed to a second loading column (Load 2), The effect of the Load 2 step was primarily to wash the second column with the buffer flowing out from the first column. In PCC run 1, the duration of the Elution 2 step was longer than for the other steps, necessitating some waiting time in the other steps.

### PCC run 1

**Table 3. Conditions for PCC run 1**

| Step | Buffer | Column volumes | Flow rate (ml/min) | Flow velocity (cm/h) | Residence time (min) |
|---|---|---|---|---|---|
| Equilibration | Equilibration buffer | 4 | 5.0 | 150 | 4 |
| Load | Sample | 0.2 | 1.8 | 54 | 11 |
| Elution 1 | Elution buffer | 0.36 | 1.8 | 54 | 11 |
| Elution 2 | Elution buffer | 1 | 5.0 | 150 | 4 |

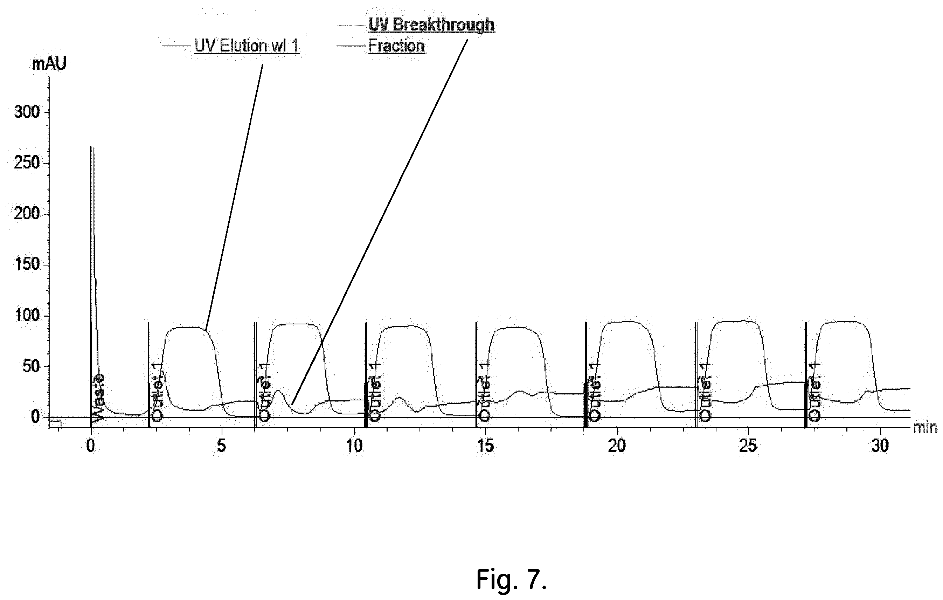

The results, as shown in Fig. 7, indicate a reasonably good performance, although with some UV breakthrough already during loading, indicating that some plasmid is eluted directly in the flowthrough.

### PCC run 2

In this experiment, the amount loaded was slightly reduced to avoid elution in the flowthrough. Further, the step times were adjusted so that all steps could be run continuously without any waiting times. As above, the equilibration step was only run once, before the actual PCC cycles.

**Table 4. Conditions for PCC run 2**

| Step | Buffer | Column volumes | Flow rate (ml/min) | Flow velocity (cm/h) | Residence time (min) |
|---|---|---|---|---|---|
| Equilibration | Equilibration buffer | 4 | 5.0 | 150 | 4 |
| Load | Sample | 0.18 | 0.9 | 27 | 22 |
| Elution 1 | Elution buffer | 0.36 | 1.8 | 54 | 11 |
| Elution 2 | Elution buffer | 1 | 5.0 | 150 | 4 |

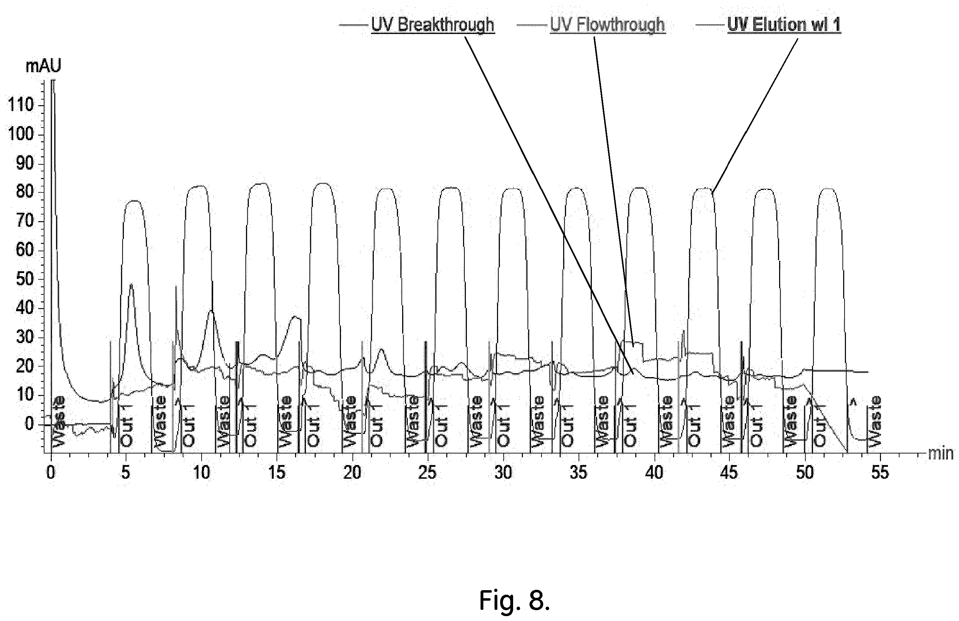

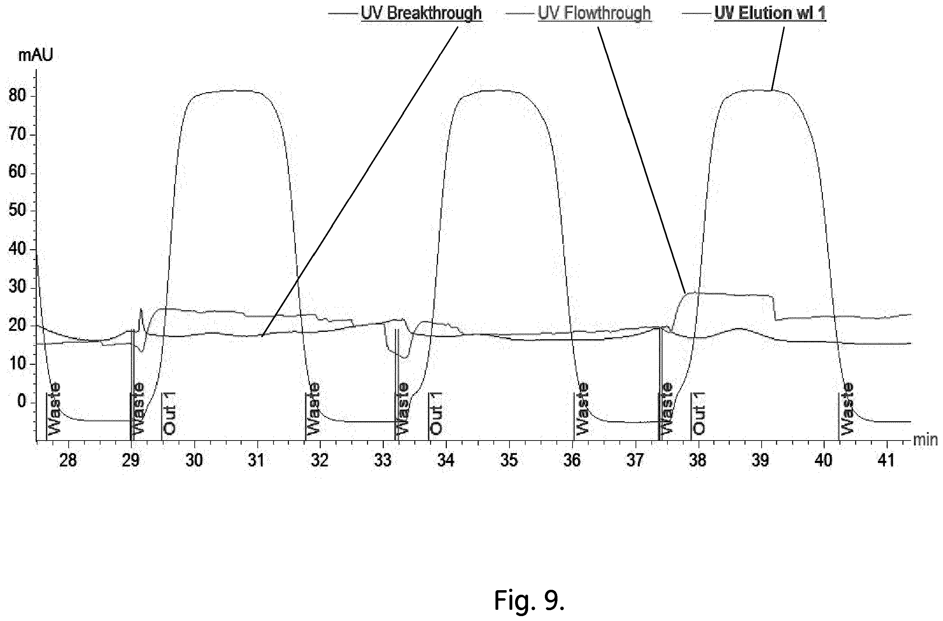

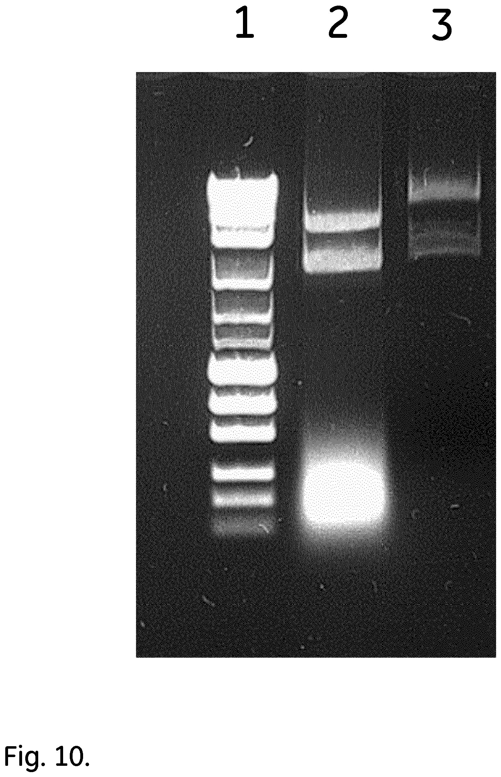

The results are shown in Figs 8 and 9, demonstrating a fully continuous separation. The electrophoresis gel in Fig. 10 shows an essentially complete removal of the low Mw RNA band from the high Mw plasmid. A simple way of expressing the productivity is to calculate the number of column volumes (CV) loaded per hour. For the single column batch experiments, at 1.67 ml/min (Run 1), loading is possible every 25 min, leading to a productivity of 0.7 CV/h. At 3.0 ml/min, the column can be loaded every 12 min, i.e. the productivity is 1.5 CV/h. In the optimised PCC example (PCC run 2), 0.18 CV can be loaded every 4.2 min, corresponding to a productivity of 2.6 CV/h, i.e. a 170% improvement over Run 2 and 370% over Run 1. A continuous three-column setup can be expected to give even higher productivities.

## Claims

1. A method of continuous separation of a plasmid from a process feed comprising a plasmid in an apparatus with at least three chromatography columns packed with particles of the same separation matrix, wherein while one chromatography column is loaded with the process feed, another chromatography column is eluted with an eluent to recover the separated plasmid, and yet another chromatography column is eluted with a further eluent to remove contaminants.

2. The method of claim 1, comprising the successive steps of:
a) conveying a process feed comprising a plasmid through a first chromatography column comprising a packed bed of separation matrix particles;
b) conveying an eluent through said first chromatography column, recovering the eluent with the plasmid after passage of the first chromatography column; and conveying said process feed through a second chromatography column packed with the same separation matrix as the first chromatography column; and
c) conveying a further eluent through said first chromatography column; conveying an eluent through said second chromatography column, recovering the eluent with the plasmid after passage of the second chromatography column; and conveying said process feed through a third chromatography column packed with the same separation matrix as the first and second chromatography columns.

3. The method of claim 2, wherein after step c), steps a)-c) are repeated for the duration of the continuous process, and wherein optionally in the repetitions:
step a) further comprises conveying a further eluent through said second chromatography column while conveying an eluent through said third chromatography column and recovering the eluent with the plasmid after passage of the third chromatography column; and
step b) further comprises conveying a further eluent through said third chromatography column.

4. The method of any one of claims 2-3, wherein a process feed or eluent is conveyed through all three columns throughout all of steps a)-c).

5. The method of claim 1, comprising the successive steps of:
a) conveying a process feed comprising a plasmid through a first chromatography column comprising a packed bed of separation matrix particles, and conveying an outflow from said first chromatography column through a second chromatography column packed with the same separation matrix as the first column;
b) conveying an eluent through said first chromatography column, recovering the eluent with the plasmid after passage of the first chromatography column; and conveying said process feed through said second chromatography column and further through a third chromatography column packed with the same separation matrix as the first and second columns;
c) conveying a further eluent through said first chromatography column; conveying an eluent through said second chromatography column, recovering the eluent with the plasmid after passage of the second chromatography column; and conveying said process feed through said third chromatography column and further through a fourth chromatography column packed with the same separation matrix as the first, second and third columns; and
d) conveying a process feed comprising a plasmid through said fourth chromatography column and conveying an outflow from said fourth chromatography column through said first chromatography column; conveying a further eluent through said second chromatography column; and conveying an eluent through said third chromatography column, recovering the eluent with the plasmid after passage of the third chromatography column.

6. The method of claim 5, wherein after step d), steps a)-d) are repeated for the duration of the continuous process, and wherein optionally in the repetitions:
step a) further comprises conveying a further eluent through said third chromatography column and conveying an eluent through said fourth chromatography column while recovering the eluent with the plasmid after passage of the fourth chromatography column; and
step b) further comprises conveying a further eluent through said fourth chromatography column.

7. The method of any one of claims 5-6, wherein a process feed or eluent is conveyed through all three columns throughout all of steps a)-d).

8. The method of any preceding claim, wherein said separation matrix particles are gel filtration particles having an exclusion limit for dextrans of less than 5000 kDa, such as of 500 kDa-5000 kDa or 500-3000 kDa.

9. The method of any preceding claim, wherein said separation matrix particles comprise a crosslinked polysaccharide, such as crosslinked agarose.

10. The method of any preceding claim, wherein:
a) said eluent comprises at least 1.5 M of a salt, such as 1.5-2.5 or 1.8-2.3 M salt;
b) the total salt concentration of said eluent is at least 1.5 M, such as 1.5-2.5 or 1.8-2.3 M; and/or
c) said eluent comprises at least 1.5 M ammonium sulfate, such as 1.5-2.5 or 1.8-2.3 M ammonium sulfate.

11. The method of any preceding claim, wherein said process feed comprises a clarified cell lysate.

12. The method of any preceding claim, comprising, before the separation, a step 0) of preparing a clarified cell lysate as the process feed, wherein step 0) optionally comprises contacting cells, such as *E. coli* cells, with alkali and a surfactant, such as SDS and, further optionally is carried out in a continuous mode.

13. The method of claim 12, wherein step 0) further comprises the addition of a hydrogen carbonate salt, such as ammonium hydrogen carbonate, optionally followed by adjustment of pH to 4-6.

14. The method of any preceding claim, further comprising, after the separation, a step e) of purifying the recovered plasmid, which optionally comprises bind-elute chromatography, such as thiophilic or hydrophobic interaction chromatography.

15. The method of claim 14, wherein step e) is operated in a continuous mode.

## Patentansprüche

1. Verfahren zur kontinuierlichen Trennung eines Plasmids aus einer ein Plasmid umfassenden Prozessbeschickung in einer Einrichtung mit mindestens drei Chromatographiesäulen, die mit Partikeln derselben Trennmatrix gepackt sind, wobei, während eine Chromatographiesäule mit der Prozessbeschickung beladen wird, eine andere Chromatographiesäule mit einem Eluenten eluiert wird, um das getrennte Plasmid zu gewinnen, und eine weitere Chromatographiesäule mit einem weiteren Eluenten eluiert wird, um Verunreinigungen zu entfernen.

2. Verfahren nach Anspruch 1, umfassend die aufeinanderfolgenden Schritte des:
a) Förderns einer Prozessbeschickung, die ein Plasmid umfasst, durch eine erste Chromatographiesäule, die ein gepacktes Bett aus Trennmatrixpartikeln umfasst;
b) Förderns eines Eluenten durch die erste Chromatographiesäule, des Gewinnens des Eluenten mit dem Plasmid nach dem Durchlaufen der ersten Chromatographiesäule; und des Förderns der Prozessbeschickung durch eine zweite Chromatographiesäule, die mit der gleichen Trennmatrix wie die erste Chromatographiesäule gepackt ist; und
c) Förderns eines weiteren Eluenten durch die erste Chromatographiesäule; des Förderns eines Eluenten durch die zweite Chromatographiesäule, des Gewinnens des Eluenten mit dem Plasmid nach dem Durchlaufen der zweiten Chromatographiesäule; und des Förderns der Prozessbeschickung durch eine dritte Chromatographiesäule, die mit der gleichen Trennmatrix wie die erste und zweite Chromatographiesäule gepackt ist.

3. Verfahren nach Anspruch 2, wobei nach Schritt c) die Schritte a)-c) für die Dauer des kontinuierlichen Prozesses wiederholt werden, und wobei wahlweise in den Wiederholungen:
Schritt a) weiter Fördern eines weiteren Eluenten durch die zweite Chromatographiesäule, während ein Eluent durch die dritte Chromatographiesäule gefördert wird, und Gewinnen des Eluenten mit dem Plasmid nach dem Durchlaufen der dritten Chromatographiesäule umfasst; und
Schritt b) weiter Fördern eines weiteren Eluenten durch die dritte Chromatographiesäule umfasst.

4. Verfahren nach einem der Ansprüche 2-3, wobei eine Prozessbeschickung oder ein Eluent durch alle drei Säulen während aller Schritte a)-c) gefördert wird.

5. Verfahren nach Anspruch 1, umfassend die aufeinanderfolgenden Schritte des:
a) Förderns einer Prozessbeschickung, die ein Plasmid umfasst, durch eine erste Chromatographiesäule, die ein gepacktes Bett von Trennmatrixpartikeln umfasst, und des Förderns eines Ausflusses aus der ersten Chromatographiesäule durch eine zweite Chromatographiesäule, die mit der gleichen Trennmatrix wie die erste Säule gepackt ist;
b) Förderns eines Eluenten durch die erste Chromatographiesäule, des Gewinnens des Eluenten mit dem Plasmid nach dem Durchlaufen der ersten Chromatographiesäule; und des Förderns der Prozessbeschickung durch die zweite Chromatographiesäule und weiter durch eine dritte Chromatographiesäule, die mit der gleichen Trennmatrix wie die erste und zweite Säule gepackt ist;
c) Förderns eines weiteren Eluenten durch die erste Chromatographiesäule; des Förderns eines Eluenten durch die zweite Chromatographiesäule, des Gewinnens des Eluenten mit dem Plasmid nach dem Durchlaufen der zweiten Chromatographiesäule; und des Förderns der Prozessbeschickung durch die dritte Chromatographiesäule und weiter durch eine vierte Chromatographiesäule, die mit der gleichen Trennmatrix wie die erste, zweite und dritte Säule gepackt ist; und
d) Förderns einer Prozessbeschickung, die ein Plasmid umfasst, durch die vierte Chromatographiesäule und des Förderns eines Ausflusses aus der vierten Chromatographiesäule durch die erste Chromatographiesäule; des Förderns eines weiteren Eluenten durch die zweite Chromatographiesäule; und des Förderns eines Eluenten durch die dritte Chromatographiesäule, des Gewinnens des Eluenten mit dem Plasmid nach dem Durchlaufen der dritten Chromatographiesäule.

6. Verfahren nach Anspruch 5, wobei nach Schritt d) die Schritte a)-d) für die Dauer des kontinuierlichen Prozesses wiederholt werden, und wobei wahlweise in den Wiederholungen:
Schritt a) weiter Fördern eines weiteren Eluenten durch die dritte Chromatographiesäule und Fördern eines Eluenten durch die vierte Chromatographiesäule umfasst, während der Eluent mit dem Plasmid nach dem Durchlaufen der vierten Chromatographiesäule gewonnen wird; und
Schritt b) weiter Fördern eines weiteren Eluenten durch die vierte Chromatographiesäule umfasst.

7. Verfahren nach einem der Ansprüche 5-6, wobei eine Prozessbeschickung oder ein Eluent durch alle drei Säulen während aller Schritte a)-d) gefördert wird.

8. Verfahren nach einem vorstehenden Anspruch, wobei die Trennmatrixpartikel Gelfiltrationspartikel sind, die eine Ausschlussgrenze für Dextran von weniger als 5000 kDa wie 500 kDa-5000 kDa oder 500-3000 kDa aufweisen.

9. Verfahren nach einem vorstehenden Anspruch, wobei die Trennmatrixpartikel ein vernetztes Polysaccharid, wie vernetzte Agarose, umfassen.

10. Verfahren nach einem vorstehenden Anspruch, wobei:
a) der Eluent mindestens 1,5 M eines Salzes umfasst, wie 1,5-2,5 oder 1,8-2,3 M Salz;
b) die Gesamtsalzkonzentration des Eluenten mindestens 1,5 M, wie 1,5-2,5 oder 1,8-2,3 M beträgt; und/oder
c) der Eluent mindestens 1,5 M Ammoniumsulfat, wie 1,5-2,5 oder 1,8-2,3 M Ammoniumsulfat, umfasst.

11. Verfahren nach einem vorstehenden Anspruch, wobei die Prozessbeschickung ein geklärtes Zelllysat umfasst.

12. Verfahren nach einem vorstehenden Anspruch, umfassend vor der Trennung einen Schritt 0) zum Herstellen eines geklärten Zelllysats als Prozessbeschickung, wobei Schritt 0) wahlweise Kontaktieren von Zellen, wie E. coli-Zellen, mit Alkali und einem Tensid, wie SDS, umfasst und weiter wahlweise in einem kontinuierlichen Modus durchgeführt wird.

13. Verfahren nach Anspruch 12, wobei Schritt 0) weiter die Zugabe eines Hydrogencarbonatsalzes, wie Ammoniumhydrogencarbonat, wahlweise gefolgt von einer Anpassung des pH-Werts auf 4-6 umfasst.

14. Verfahren nach einem vorstehenden Anspruch, weiter umfassend nach der Trennung einen Schritt e) zum Reinigen des gewonnenen Plasmids, der wahlweise eine Binden-Eluieren-Chromatographie, wie die thiophile oder hydrophobe Wechselwirkungschromatographie, umfasst.

15. Verfahren nach Anspruch 14, wobei Schritt e) in einem kontinuierlichen Modus durchgeführt wird.

## Revendications

1. Procédé de séparation continue d'un plasmide à partir d'une charge de traitement comprenant un plasmide dans un appareil comportant au moins trois colonnes de chromatographie garnies de particules de la même matrice de séparation, dans lequel tandis qu'une colonne de chromatographie est chargée avec la charge de traitement, une autre colonne de chromatographie est éluée avec un éluant pour récupérer le plasmide séparé, et encore une autre colonne de chromatographie est éluée avec un autre éluant pour éliminer les contaminants.

2. Procédé selon la revendication 1, comprenant les étapes successives de :
a) transport d'une charge de traitement comprenant un plasmide à travers une première colonne de chromatographie comprenant un lit garni de particules de matrice de séparation ;
b) transport d'un éluant à travers ladite première colonne de chromatographie, récupération de l'éluant avec le plasmide après passage de la première colonne de chromatographie ; et transport de ladite charge de traitement à travers une deuxième colonne de chromatographie garnie de la même matrice de séparation que la première colonne de chromatographie ; et
c) transport d'un autre éluant à travers ladite première colonne de chromatographie ; transport d'un éluant à travers ladite deuxième colonne de chromatographie, récupération de l'éluant avec le plasmide après passage de la deuxième colonne de chromatographie ; et transport de ladite charge de traitement à travers une troisième colonne de chromatographie garnie de la même matrice de séparation que les première et deuxième colonnes de chromatographie.

3. Procédé selon la revendication 2, dans lequel après l'étape c), les étapes a)-c) sont répétées pendant la durée du processus continu, et dans lequel éventuellement dans les répétitions :
l'étape a) comprend en outre le transport d'un autre éluant à travers ladite deuxième colonne de chromatographie tout en transportant un éluant à travers ladite troisième colonne de chromatographie et la récupération de l'éluant avec le plasmide après passage de la troisième colonne de chromatographie ; et
l'étape b) comprend en outre le transport d'un autre éluant à travers ladite troisième colonne de chromatographie.

4. Procédé selon l'une quelconque des revendications 2-3, dans lequel une charge de traitement ou un éluant est transporté à travers toutes les trois colonnes tout au long de toutes les étapes a) à c).

5. Procédé selon la revendication 1, comprenant les étapes successives de :
a) transport d'une charge de traitement comprenant un plasmide à travers une première colonne de chromatographie comprenant un lit garni de particules de matrice de séparation, et transport d'un écoulement sortant depuis ladite première colonne de chromatographie à travers une deuxième colonne de chromatographie garnie de la même matrice de séparation que la première colonne ;
b) transport d'un éluant à travers ladite première colonne de chromatographie, récupération de l'éluant avec le plasmide après passage de la première colonne de chromatographie ; et transport de ladite charge de traitement à travers ladite deuxième colonne de chromatographie et ensuite à travers une troisième colonne de chromatographie garnie de la même matrice de séparation que les première et deuxième colonnes ;
c) transport d'un autre éluant à travers ladite première colonne de chromatographie ; transport d'un éluant à travers ladite deuxième colonne de chromatographie, récupération de l'éluant avec le plasmide après passage de la deuxième colonne de chromatographie ; et transport de ladite charge de traitement à travers ladite troisième colonne de chromatographie et ensuite à travers une quatrième colonne de chromatographie garnie de la même matrice de séparation que les première, deuxième et troisième colonnes ; et
d) transport d'une charge de traitement comprenant un plasmide à travers ladite quatrième colonne de chromatographie et transport d'un écoulement sortant depuis ladite quatrième colonne de chromatographie à travers ladite première colonne de chromatographie ; transport d'un autre éluant à travers ladite deuxième colonne de chromatographie ; et transport d'un éluant à travers ladite troisième colonne de chromatographie, récupération de l'éluant avec le plasmide après passage de la troisième colonne de chromatographie.

6. Procédé selon la revendication 5, dans lequel après l'étape d), les étapes a) à d) sont répétées pendant la durée du processus continu, et dans lequel éventuellement dans les répétitions :
l'étape a) comprend en outre le transport d'un autre éluant à travers ladite troisième colonne de chromatographie et le transport d'un éluant à travers ladite quatrième colonne de chromatographie tout en récupérant l'éluant avec le plasmide après passage de la quatrième colonne de chromatographie ; et
l'étape b) comprend en outre le transport d'un autre éluant à travers ladite quatrième colonne de chromatographie.

7. Procédé selon l'une quelconque des revendications 5-6, dans lequel une charge de traitement ou un éluant est transporté à travers toutes les trois colonnes tout au long de toutes les étapes a) à d).

8. Procédé selon une quelconque revendication précédente, dans lequel lesdites particules de matrice de séparation sont des particules de filtration sur gel présentant une limite d'exclusion pour les dextranes inférieure à 5 000 kDa, telle que 500 kDa-5 000 kDa ou 500-3 000 kDa.

9. Procédé selon une quelconque revendication précédente, dans lequel lesdites particules de matrice de séparation comprennent un polysaccharide réticulé, tel que l'agarose réticulé.

10. Procédé selon une quelconque revendication précédente, dans lequel :
a) ledit éluant comprend au moins 1,5 M d'un sel, tel que 1,5-2,5 ou 1,8-2,3 M de sel ;
b) la concentration totale en sel dudit éluant est d'au moins 1,5 M, telle que 1,5-2,5 ou 1,8-2,3 M ; et/ou
c) ledit éluant comprend au moins 1,5 M de sulfate d'ammonium, tel que 1,5-2,5 ou 1,8-2,3 M de sulfate d'ammonium.

11. Procédé selon une quelconque revendication précédente, dans lequel ladite charge de traitement comprend un lysat cellulaire clarifié.

12. Procédé selon une quelconque revendication précédente, comprenant, avant la séparation, une étape 0) de préparation d'un lysat cellulaire clarifié en tant que charge de traitement, dans lequel l'étape 0) comprend éventuellement la mise en contact de cellules, telles que des cellules d'*E*. *coli*, avec un alcali et un tensioactif, tel que SDS et, en outre éventuellement, est effectuée en mode continu.

13. Procédé selon la revendication 12, dans lequel l'étape 0) comprend en outre l'ajout d'un sel d'hydrogénocarbonate, tel que l'hydrogénocarbonate d'ammonium, éventuellement suivi d'un ajustement du pH à 4-6.

14. Procédé selon une quelconque revendication précédente, comprenant en outre, après la séparation, une étape e) de purification du plasmide récupéré, qui comprend éventuellement une chromatographie de liaison-élution, telle qu'une chromatographie d'interaction thiophile ou hydrophobe.

15. Procédé selon la revendication 14, dans lequel l'étape e) est effectuée en mode continu.
